# EUROPEAN PATENT SPECIFICATION

(11) **EP 4 284 350 B1**
(45) Date of publication and mention of the grant of the patent: **20.05.2026**
(21) Application number: 22703306.5
(22) Date of filing: 24.01.2022
(51) Int. Cl.: A61K 31/05, A61K 9/00, A61K 9/06, A61K 9/107, A61K 47/44, A61K 47/46, A61K 47/10, A61K 47/14, A61K 47/32, A61K 47/18, A61K 47/08, A61K 47/38, A61K 47/02, A61K 47/24, A61K 47/12, A61P 17/00, A61P 29/00

(54) **COMPOSITIONS CONTAINING CANNABIDIOL AND BROCCOLI SEED OIL**
ZUSAMMENSETZUNGEN MIT CANNABIDIOL UND BROKKOLISAMENÖL
COMPOSITIONS CONTENANT DU CANNABIDIOL ET DE L'HUILE DE GRAINES DE BROCOLI

(30) Priority: 26.01.2021 US 202163141721 P
(43) Date of publication of application: 06.12.2023
(73) Proprietor: Société des Produits Nestlé S.A., 1800 Vevey (CH)
(72) Inventor: VINYES PARES, Gerard, 08328 Alella (ES); RAN-RESSLER, Rinat, Bridgewater, New Jersey 08807 (US); RAU, Stefanie, 82140 Olching (DE)
(74) Representative: Gordon, Kirsteen Helen
(86) International application number: PCT/EP2022/051415
(87) International publication number: WO 2022/161883

(56) References cited:
- WO-A1-2013/149323
- CA-A1- 3 138 630
- US-A1- 2019 192 658
- LISA ROUGH: "7 Must-Try Cannabis Topicals for Those Scorching Summer Days | Leafly", 3 July 2017 (2017-07-03), pages 1 - 9, XP055634347, Retrieved from the Internet <URL:https://www.leafly.com/news/strains-products/cannabis-topicals-for-summer> [retrieved on 20191021]

## Description

### BACKGROUND

The present disclosure relates to compositions comprising cannabidiol and broccoli seed oil, and further describes methods of making such compositions and using such compositions, preferably for topical application.

Cannabidiol (CBD) is one of the most abundant cannabinoids found in the plant Cannabis Sativa, considered to have anti-inflammatory properties and of interest in treating a variety of skin diseases, inflammatory disorders or other dermatology conditions.

### SUMMARY

An issue with cannabidiol can be that its lyphopylic nature results in very low skin penetration, thereby limiting its bioefficacy. Current clinical applications of topical CBD have been limited by sub-optimal formulations, hence decreasing therapeutic penetration and absorption by the skin, and most of CBD remains through the stratum corneum (SC), the main barrier to penetration of the skin in its uppermost layer. Some solutions have been already proposed as alternatives, like using nano-particles to increase its penetration, yet these are not always accepted in the cosmetic application.

As detailed later herein, it was demonstrated that a specific emulsion of CBD (cannabidiol) and botanicals (; comfrey; camphor; menthol; aloe vera) with broccoli seed oil, Isoamyl Laurate and cellulose was able to increase CBD penetration in the skin. An *ex-vivo* study with human skin samples demonstrated that the active formulation provided significantly more penetration than a control, in the epidermis and dermis skin layers. A non-limiting example of a suitable concentration of CBD in the product can be about 0.5 wt.%, but other embodiments have higher or lower concentrations, such as 0.05 wt.% to 5 wt.% CBD. In some embodiments, at least a portion of the CBD can be synthetic CBD. Additionally or alternatively, at least a portion of the CBD can be botanically derived CBD.

US2019192658A1 describes a topical pain relief composition that includes a matrix including a water soluble active ingredient, a water insoluble active ingredient, and purified water. A first-combination of phoenoxyethanol, caprynyl glycol and, sorbic acid is described. Further, a second-combination is provided of oryza sativa bran extract, rosmarinus officinalis leaf extract, helianthus anuus extract and, tocopherol.

CA3138630 describes a versatile cannabinoid stock. The cannabinoid stock is a substantially homogeneous concentrated stable cannabinoid emulsion characterized as a cannabinoid load capacity carrier that comprises emulsified particles of stabilized cannabinoid/lipid. In a embodiment CA'630 describes a pain relief gel comprising 150mg CBD, Water, Lecigel (Sodium acrylates copolymer and lecithin), Glycerin, Allantoin, Sodium Hyaluronate, CBD Stock lipid, Camphor, Allantoin, Menthol, Vitamin E Acetate (Tocopherol acetate), Arnica Extract GL, Boswellia Extract GL, Aloe Veral Gel 10x (Ale Barbadensis Leaf Juice) AND Euxyl PE 9010.

Additional features and advantages are described herein and will be apparent from the following Figures and Detailed Description.

### BRIEF DESCRIPTION OF DRAWINGS

**FIG. 1** is a non-limiting example of a suitable formulation for one or more embodiments disclosed herein and which was experimentally tested in the experimental example disclosed herein.
**FIG. 2** shows a comparative formulation lacking broccoli oil, aloe vera and isoamyl laurate and which was experimentally tested in the experimental example disclosed herein.
**FIGS. 3-10** set forth results from the experimental example disclosed herein.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number. All numerical ranges herein should be understood to include all integers, whole or fractions, within the range.

As used in this disclosure and the appended claims, the singular forms "a," "an" and "the" include plural referents unless the context clearly dictates otherwise. Thus, for example, reference to "a botanical compound" or "the botanical compound" includes both a single botanical compound and also two or more botanical compounds.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" further includes a disclosure of embodiments "consisting essentially of" and "consisting of" the components identified.

The terms "at least one of" and "and/or" used in the respective context of "at least one of X or Y" and "X and/or Y" should be interpreted as "X," or "Y," or "X and Y." For example, "at least one of isoamyl laurate or cellulose" should be interpreted as "isoamyl laurate without cellulose," or "cellulose without isoamyl laurate," or "both isoamyl laurate and cellulose."

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently, preferably means that the conditions are caused by the same underlying condition, and most preferably means that one of the identified conditions is caused by the other identified condition.

"Prevention" includes reduction of risk, frequency and/or severity of a condition or disorder. The terms "treatment" and "treat" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The terms "treatment" and "treat" do not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment" and "treat" are also intended to include the potentiation or otherwise enhancement of one or more primary prophylactic or therapeutic measures. As non-limiting examples, a treatment can be performed by a patient, a caregiver, a doctor, a nurse, or another healthcare professional.

As used herein, a prophylactically or therapeutically "effective amount" is an amount that prevents a deficiency, treats a disease or medical condition in an individual, or, more generally, reduces symptoms, manages progression of the disease, or provides a nutritional, physiological, or medical benefit to the individual.

The term "unit dosage form," as used herein, refers to physically discrete units suitable as unitary dosages for human and animal subjects, each unit containing a predetermined quantity of the composition disclosed herein in an amount sufficient to produce the desired effect, in association with a pharmaceutically acceptable diluent, carrier or vehicle. The specifications for the unit dosage form depend on the particular compounds employed, the effect to be achieved, and the pharmacodynamics associated with each compound in the host.

A "subject" or "individual" is a mammal, preferably a human.

### Embodiments

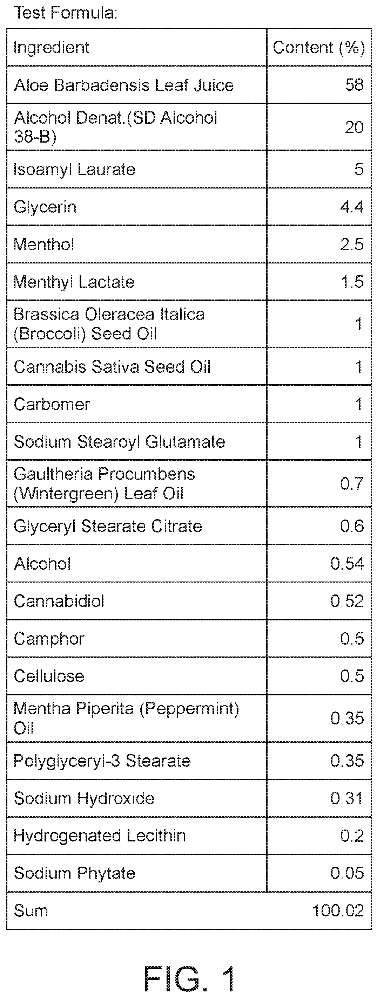

The present disclosure relates to increasing penetration of cannabidiol (CBD) in the skin of an individual. FIG. 1 shows a non-limiting example of a formulation suitable for one or more embodiments disclosed herein. The present invention discloses a composition formulated for increased penetration of cannabidiol (CBD) in the skin of an individual upon topical administration of the composition to the skin of the individual, wherein the composition consists essentially of the CBD, broccoli seed oil, ethanol, aloe vera, and isoamyl laurate. The disclosure relates to topically administering the composition to the skin of the individual. The composition may further comprise a botanical compound. The botanical compound can be selected from the group consisting of arnica, comfrey, camphor, menthol, and combinations thereof. In some embodiments, the composition further comprises cellulose. In some embodiments, the composition further comprises at least one emulsifier selected from the group consisting of sodium stearoyl glutamate, Glyceryl Stearate Citrate, Polyglyceryl-3 stearate, Lecithin, and combinations thereof. In some embodiments, the increasing of the penetration of CBD in the skin of the individual comprises increasing penetration of the CBD into the epidermis.

The CBD is preferably about 0.05 wt.% to about 5 wt.% of the composition, more preferably about 0.1 wt.% to about 2.0 wt.% of the composition, for instance about 0.1 wt.% to about 1.0 wt.% of the composition. In an embodiment the CBD is about 0.5 wt.% of the composition. The broccoli seed oil is preferably about 0.2 wt.% to about 20 wt.% of the composition, more preferably about 0.5 wt.% to about 5.0 wt.% of the composition, for instance about 0.5 wt.% to about 2.0 wt.% of the composition. In an embodiment the broccoli seed oil is about 1.0 wt.% of the composition. In an embodiment the composition comprises CBD at about 0.5 wt.% of the composition and broccoli oil at about 1.0 wt. % of the composition.

In some embodiments, the composition can comprise aloe vera at about 50.0 wt.% to about 60.0 wt.% of the composition and/or alcohol at about 15.0 wt.% to about 25.0 wt.% of the composition.

The composition consists essentially of, or consists of: the CBD, the broccoli seed oil, ethanol, aloe vera, and isoamyl laurate. In such embodiments, the composition can comprise aloe vera at about 50.0 wt.% to about 60.0 wt.% of the composition and/or alcohol at about 15.0 wt.% to about 25.0 wt.% of the composition.

In various embodiments, the composition can comprise aloe vera at about 50.0 wt.% to about 60.0 wt.% of the composition and/or ethanol at about 15.0 wt.% to about 25.0 wt.% of the composition.

In some embodiments, at least a portion of the CBD is synthetic CBD, including for example, an entirety of the CBD being synthetic CBD in some particular embodiments. Additionally or alternatively, at least a portion of the CBD is botanically derived CBD, including for example, an entirety of the CBD being botanically derived CBD in some particular embodiments.

At least a portion of the broccoli seed oil may be Brassica Oleracea Italica Seed Oil.

The composition may be administered in a dose comprising an amount of the CBD effective for treating, reducing a severity of, reducing an incidence of, reducing a frequency of, or preventing at least one condition selected from the group consisting of a skin disease, another dermatology condition, an inflammatory disorder, anxiety, insomnia, chronic pain, and combinations thereof.

The composition is administered in a dose comprising an amount of the broccoli seed oil effective for increasing penetration of the CBD in the skin, relative to a reference composition lacking the broccoli seed oil but otherwise identically formulated.

The composition according to the invention can optionally further comprise an additional component, for example one or more of (i) a skin care agent such as one or more of Aloe Barbadensis Leaf Juice, Symphytum Officinale Root Extract, or Symphytum Officinale Root Extract; (ii) a solvent such as one or more of water, denatured alcohol, or glycerin; (iii) a masking agent such as one or more of menthol, menthyl lactate, or camphor; (iv) an emollient such as one or more of cannabis sativa seed oil, or Helianthus Annuus (Sunflower) Seed Oil; (v) a moisturizer such as glycerin; (vi) an emulsion stabilizer such as carbomer or xanthan gum; (vii) a stabilizer such as cellulose; (viii) an emulsifier such as sodium stearoyl glutamate, Glyceryl Stearate Citrate; Polyglyceryl-3 stearate or Lecithin; (ix) a fragrance such as one or more of Gaultheria Procumbens (Wintergreen) Leaf Oil, Mentha Piperita (Peppermint) Oil, limonene, or linalool; (x) a buffer such as sodium hydroxide; (xi) an antioxidant such as tocopherol; (xii) a cosmetic dye such as caramel; (xiii) sodium phytate; and/or (xiv) Amica Montana Flower Extract. Isoamyl laurate is an emollient with very good spreading and absorption behavior that thereby provides better penetration of the CBD into the skin; and cellulose, which is a stabilizer and also achieves a mattifying effect on the skin.

The composition is formulated for a topical administration and thus can be aqueous, hydroalcoholic or oily solutions, solution-type dispersions or lotion or serum-type dispersions, emulsions having a liquid or semi-liquid consistency of milk-type, suspensions or emulsions of cream-type, aqueous or anhydrous gel, microemulsions, microcapsules, microparticles, or vesicular dispersions of ionic and/or nonionic type.

These compositions can constitute cleansing, protection, treatment or care creams for the face, hands, feet, the large anatomical folds or the body, for example day creams, night creams, make-up removers, basic foundation creams, anti-sun creams, makeup products like fluid foundations, make-up remover milks, body milks for protection or care, after-sun milks, lotions, gels or foams for skin care, like cleansing or disinfection lotions, anti-sun lotions, artificial suntan lotions, compositions for the bath, deodorant compositions containing a bactericidal agent, gels or after-shave lotions, depilatory creams, or compositions against insect bites.

The method can be implemented by applying any of the compositions disclosed herein according to the conventional technique of the use of these compositions. For example, applications of creams, gels, sera, lotions, make-up remover milks or after-sun compositions to the skin or dry hair; application of a hair lotion or shampoo on wet hair; or application of toothpaste to the gums.

The composition can be administered at least one day per week, preferably at least two days per week, more preferably at least three or four days per week (e.g., every other day), most preferably at least five days per week, six days per week, or seven days per week. The time period of administration can be at least one week, preferably at least one month, more preferably at least two months, most preferably at least three months, for example at least four months. In an embodiment, dosing is at least daily; for example, a subject may receive one or more doses daily. In other embodiments, the administration occurs until no detectable symptoms of the medical condition remain. In specific embodiments, the administration occurs until a detectable improvement of at least one symptom occurs and, in further cases, continues to remain ameliorated.

Herein is also described a method of making a composition formulated for increased penetration of cannabidiol (CBD) in the skin of an individual upon topical administration of the composition to the individual. The method can comprise adding broccoli seed oil to the CBD to thereby form at least a portion of the composition.

Said method further comprises adding a botanical compound being aloe vera to at least one of the broccoli seed oil, the CBD or the composition. A further botanical compound can be selected from the group consisting of arnica, comfrey, camphor, menthol, and combinations thereof.

Said method further comprises adding at least isoamyl laurate or further cellulose to at least one of the broccoli seed oil, the CBD or the composition.

Said method may further comprise adding at least one emulsifier selected from the group consisting of sodium stearoyl glutamate, Glyceryl Stearate Citrate, Polyglyceryl-3 stearate, Lecithin, and combinations thereof to at least one of the broccoli seed oil, the CBD or the composition.

In some embodiments, the CBD is about 0.5 wt.% of the composition, and/or the broccoli seed oil is about 1.0 wt.% of the composition.

In some embodiments, at least a portion of the CBD is synthetic CBD. Additionally or alternatively, at least a portion of the CBD is botanically derived CBD.

In some embodiments, the composition is a unit dosage form comprising a therapeutically or prophylactically effective amount of the CBD. The unit dosage form comprises an amount of the broccoli seed oil effective for increasing penetration of the CBD in the skin, relative to a reference composition lacking the broccoli seed oil but otherwise identically formulated.

In some embodiments, at least a portion of the broccoli seed oil is Brassica Oleracea Italica Seed Oil.

### EXAMPLE

The following non-limiting example presents experimental data for embodiments according to the present disclosure.

### INTRODUCTION

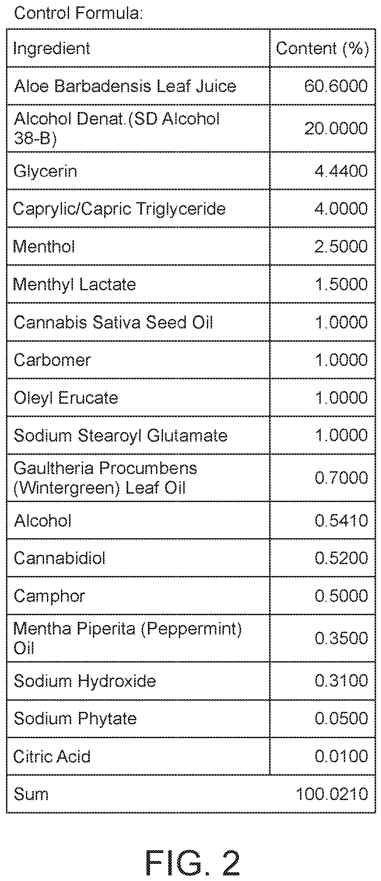

An analytical method was performed for the assay of cannabidiol into the skin (Ex *vivo*). The objective of the study was to evaluate the *ex-vivo* cutaneous distribution of the Cannabidiol (CBD), contained in 2 formulations. The study is conducted on human skin explants. The formulation shown in **FIG. 1** was tested as representative of an embodiment according to the present disclosure ("F2 Wobecare CBD Active cream variante 7"). The formulation shown in **FIG. 2** was used as a comparative example and was lacking broccoli oil, aloe vera and isoamyl laurate ("F1 Comparison").

### MATERIALS AND METHODS

### 1. Biological model

Human abdominal skin samples were excised during surgical operations. Skins were examined upon reception and only those with a homogeneous appearance and no macroscopic defaults were retained. The skin samples were obtained from the plastic surgery department of a clinic following "abdominoplasty" surgical procedures.

This study was performed on freezed human skin in Tours (France) in accordance to the guideline of the OECD N° 428 Skin Absorption.

### 2. Ex vivo Phase

Skin specimens from one donor were thawed at room temperature for 15 minutes and cleaned with PBS. The integrity of the skin barrier of each explant was controlled by measuring the transepidermal water loss (TEWL) using a Vapometer. The thickness of each explant was measured in five different places using a micrometer.

As the TEWL values measured for the 15 explants were around 6.4 g.m-².h-1, the skin barrier of the skin explants is considered intact and appropriate for use in this study. The skin explants were placed in Franz diffusion cells with a diffusion area of approximately 2 cm⁻² and a receiver chamber volume of approximately 14.5 mL were used.

The dermis was in contact with the receptor compartment and the Stratum corneum with the donor compartment. Clamps were used to hold both compartments together and thus ensure that the system was leak-proof. The receptor compartments were filled with a PBS + Brij O20 at 0.1% solution. Particular care was taken to prevent the formation of air bubbles beneath the skin explants (dermis interface) in the receptor liquid.

The cells were placed in the oven for 1 hour to obtain a hydration level and equivalent thermal balance for all skin explants.

At the end of the equilibration stage, the formulation was carefully applied to the surface of the explants using a positive displacement micropipette.

**10 mg (5 mg.cm⁻²) of formulation was applied to the skin surface.**

The diffusion cells were placed on a magnet stirrer in the oven at 32°C with a humidity of 50% ±5%. The stirring speed of the receiver liquid was set at 400 U.min-1.

The duration of the exposure time to formulations was set to 24 h. At the end this duration, the explants were removed from the oven to proceed to the sampling phase of the various skin layers and receiver liquid which was stored at -20°C until analytical dosage.

For each explant, the samples were collected as follows:
- Recovery of the non-absorbed fraction of active and cleaning procedure (PN):
   the surface of the skin was cleaned using 500µL of SLS and massage with 1 cotton swab,
   followed by 10 suction-discharge of 500µL SLS,
   rinsing with 3 time of 500µL of water suction discharge,
   drying with 1 dry cotton swab,
   the 2 cotton swabs and liquids were placed in a 15 mL Falcon tube for analysis.
- Recovery of the stratum corneum (SC): strips (n=3) were realized using D-squam discs and placed in a 15 mL Falcon tube.
- Recovery of the Epidermis (Ep):
   the skin was placed on a hot plate for 15 seconds at 65°C,
   the epidermis was gently separated from the dermis using a forceps,
   the epidermis was placed in a 15 mL Falcon tube.
- Recovery of the Dermis (D): Dermis was placed in a 15 mL Falcon tube.
- Recovery of Receiver Liquid: 10 mL fraction of the receiver liquid was placed in a 15 mL Falcon tube which was stored for analysis.

The different matrices were stored at -80°C:
15 cleaning procedure (PN)
15 stratum corneum (SC)
15 epidermis (Ep)
15 dermis (Dm)
15 receiver liquids (LR)

### 3. Analytical method

The following parameters were retained:
*Column:* Kinetex C18, 150 x 4.6 mm; 2.6 µm
*Column temperature:* 30°C
*Injection volume:* 20 µl
*Mobile phase:* Water/formic ac. 0.1% (Phase A) & Methanol/formic ac. 0.1% (Phase B)
*Flow rate:* 0.5 ml/min
*Elution mode:* Gradient 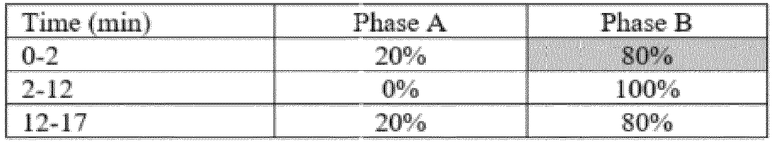
*Run time:* 17 min
*Detection mode:* ESI
*Polarity:* Positive
*Acquisition mode:* Single Ion Monitoring (SIM)
*Acquisition ion:* 315 amu for CBD and 318 amu for CBD D3

The following methodology was applied to extract CBD from test item:
Step 1: Weight the test item (100 mg).
Step 2: Dilute the cream into 100 ml of diluant solution (Phase B).
Step 3: Diluted the work solution with a solution of internal standard and analyze the sample.

The following methodology was applied to extract CBD from Epidermis:
Step 1: Tubes containing the samples were thawed at ambient temperature.
Step 2: Introduce the totality of the epidermis into the tube.
Step 3: Skin was extracted using diluant solution (Phase B) with a ball mill for disintegration (Retsch) at 30 hz for 10 min and centrifuge.
Step 4: Diluted the supernatant with a solution of internal standard and analyze the sample.

The following methodology was applied to extract CBD from Dermis:
Step 1: Tubes containing the samples were thawed at ambient temperature.
Step 2: Weight the dermis (100 mg). Cut the dermis in different slices.
Step 3: Skin was extracted using diluant solution (Phase B) with a UltraTurax^{®} followed with a ball mill for disintegration (Retsch) at 30 hz for 10 min and centrifuge.
Step 4: Diluted the supernatant with a solution of internal standard and analyze the sample.

### Results

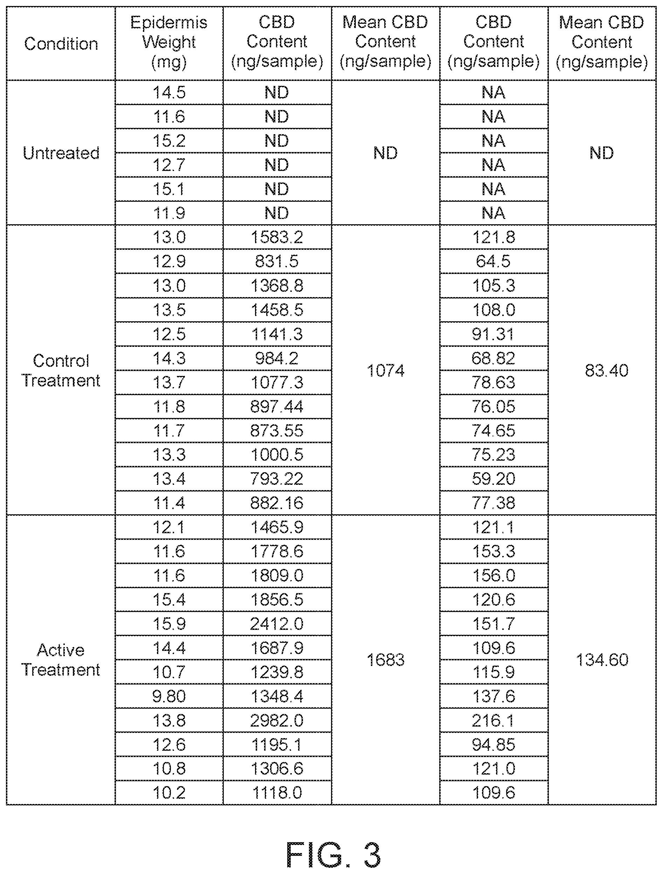

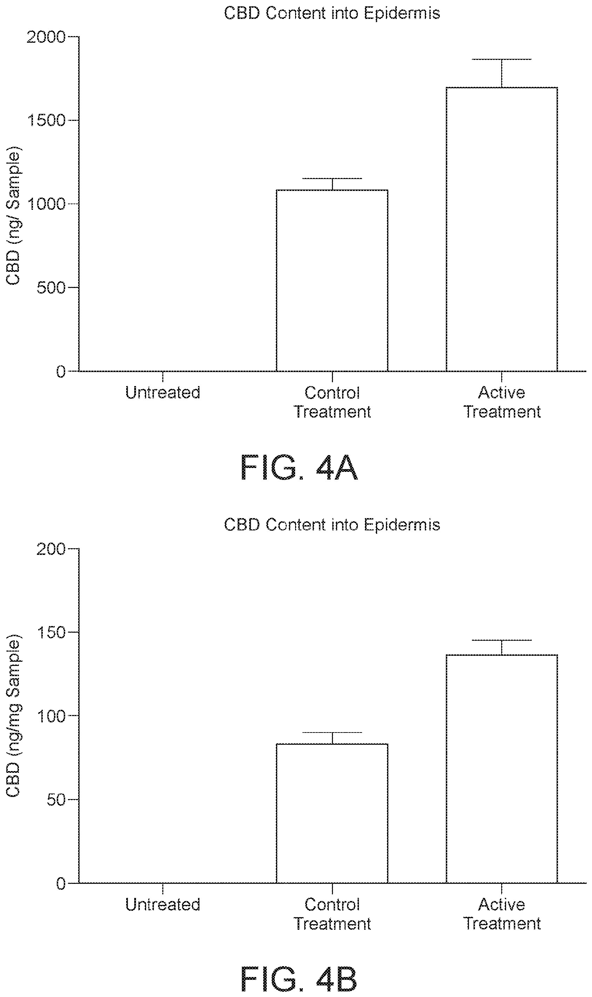

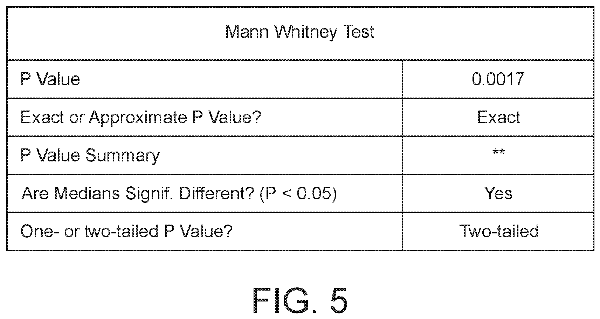

Two trial runs were performed, and each trial run included control treatment ("F1 Comparison") and active treatment ("F2 Variante 7"). Compilation results obtained on epidermis were expressed in ng/sample and in ng/mg taking account the weight of epidermis (**FIG. 3**). CBD was quantified into the epidermis that testified a penetration into the skin. The CBD content is higher for the active treatment ("F2 Variante 7") condition (the formulation representative of an embodiment according to the present disclosure). Illustrations are presented in **FIGS. 4A** and **4B****.** As shown in **FIG. 5****,** a statistical test was performed to compare each condition (Mann Whitney test).

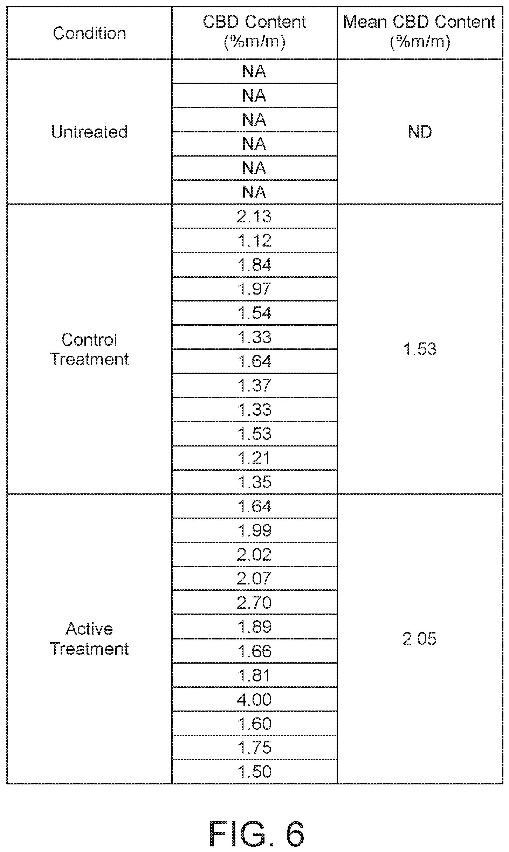

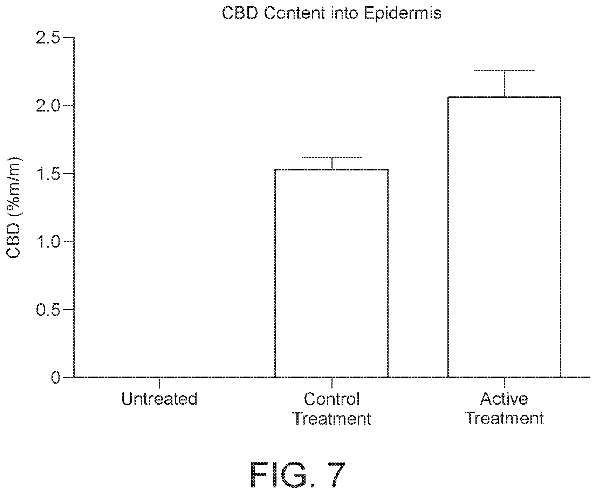

Furthermore, results obtained on epidermis were expressed in %m/m taking account of the cannabidiol content into the test item (**FIG. 6**), illustration presented in **FIG. 7****.**

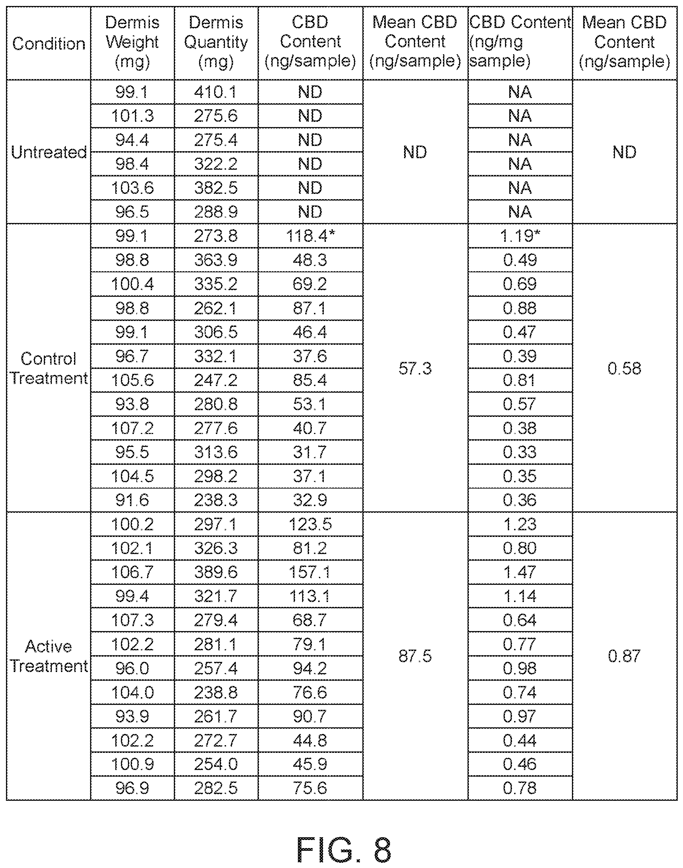

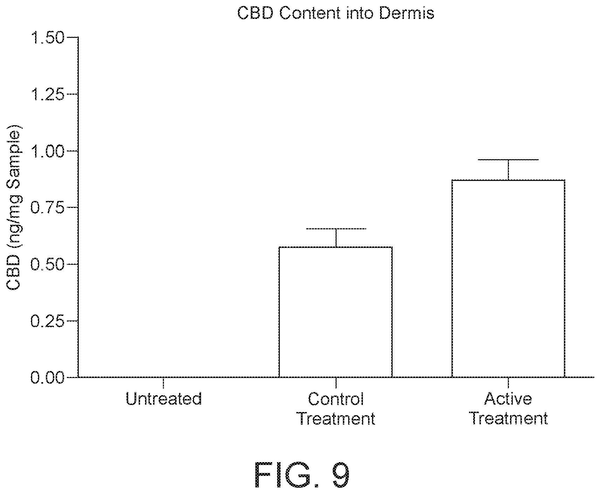

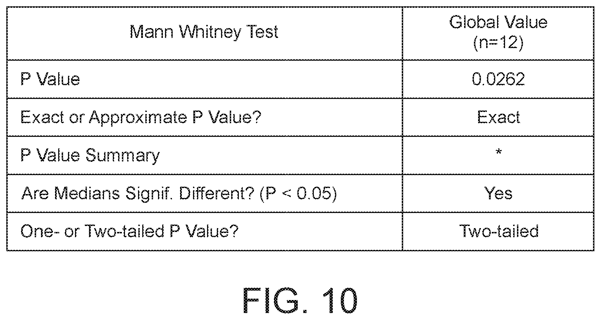

Results obtained on dermis were expressed in ng/sample and in ng/mg taking account the weight of dermis (**FIG. 8**). CBD was quantified into the dermis that testified a penetration into the skin. The CBD content is higher for the active treatment ("F2 Variante 7") condition (the formulation representative of an embodiment according to the present disclosure). Illustration is presented in **FIG. 9****.** As shown in **FIG. 10****,** a statistical test was performed to compare each condition (Mann Whitney test).

### CONCLUSION

The objective of the study was to evaluate the *ex-vivo* cutaneous distribution of the Cannabidiol (CBD), contained in 2 formulations. The study was conducted on human skin explants (2 runs).

CBD was quantified into the epidermis that testified a penetration into the skin. The CBD content is higher for the active treatment ("F2 Variante 7") condition (the formulation representative of an embodiment according to the present disclosure).

CBD was quantified into the dermis that testified a penetration into the skin. The CBD content is higher for the active treatment ("F2 Variante 7") condition (the formulation representative of an embodiment according to the present disclosure).

## Claims

1. A composition formulated for increased penetration of cannabidiol (CBD) in the skin of an individual upon topical administration of the composition to the skin of the individual, wherein the composition consists essentially of the CBD, broccoli seed oil, ethanol, aloe vera, and isoamyl laurate.

2. The composition of claim 1, wherein the ethanol is 15.0 wt.% to 25.0 wt.% of the composition, and the aloe vera is 50.0 wt.% to 60.0 wt.% of the composition.

3. The composition of claim 1 or 2, wherein at least a portion of the CBD is synthetic CBD.

4. The composition of any preceding claim, wherein at least a portion of the CBD is botanically derived CBD.

5. The composition of any preceding claim, wherein the CBD is 0.05 wt.% to 5.0 wt.%, preferably 0.5 wt.%, of the composition.

6. The composition of any preceding claim, wherein the broccoli seed oil is 0.2 wt.% to 20 wt.%, preferably 1.0 wt.%, of the composition.

## Patentansprüche

1. Zusammensetzung, die für eine erhöhte Penetration von Cannabidiol (CBD) in die Haut eines Individuums bei einer topischen Verabreichung der Zusammensetzung an die Haut des Individuums formuliert ist, wobei die Zusammensetzung im Wesentlichen aus dem CBD, Brokkolisamenöl, Ethanol, Aloe Vera und Isoamyllaurat besteht.

2. Zusammensetzung nach Anspruch 1, wobei das Ethanol 15,0 Gew.-% bis 25,0 Gew.-% der Zusammensetzung beträgt und die Aloe Vera 50,0 Gew.-% bis 60,0 Gew.-% der Zusammensetzung beträgt.

3. Zusammensetzung nach Anspruch 1 oder 2, wobei mindestens ein Anteil des CBD ein synthetisches CBD ist.

4. Zusammensetzung nach einem der vorstehenden Ansprüche, wobei mindestens ein Anteil des CBD ein botanisch gewonnenes CBD ist.

5. Zusammensetzung nach einem der vorstehenden Ansprüche, wobei das CBD 0,05 Gew.-% bis 5,0 Gew.-%, vorzugsweise 0,5 Gew.-%, der Zusammensetzung beträgt.

6. Zusammensetzung nach einem der vorstehenden Ansprüche, wobei das Brokkolisamenöl 0,2 Gew.-% bis 20 Gew.-%, vorzugsweise 1,0 Gew.-%, der Zusammensetzung beträgt.

## Revendications

1. Composition formulée pour une pénétration accrue du cannabidiol (CBD) dans la peau d'un individu lors de l'administration topique de la composition sur la peau de l'individu, dans laquelle la composition est constituée essentiellement de CBD, d'huile de graines de brocoli, d'éthanol, d'aloe vera et de laurate d'isoamyle.

2. Composition selon la revendication 1, dans laquelle l'éthanol représente 15,0 % en poids à 25,0 % en poids de la composition, et l'aloe vera représente 50,0 % en poids à 60,0 % en poids de la composition.

3. Composition selon la revendication 1 ou 2, dans laquelle au moins une partie du CBD est du CBD synthétique.

4. Composition selon l'une quelconque revendication précédente, dans laquelle au moins une partie du CBD est du CBD d'origine botanique.

5. Composition selon l'une quelconque revendication précédente, dans laquelle le CBD représente 0,05 % en poids à 5,0 % en poids, de préférence 0,5 % en poids, de la composition.

6. Composition selon l'une quelconque revendication précédente, dans laquelle l'huile de graines de brocoli représente 0,2 % en poids à 20 % en poids, de préférence 1,0 % en poids, de la composition.
